# FASCICULE DE BREVET EUROPEEN

(11) **EP 2 598 523 B1**
(45) Date de publication et mention de la délivrance du brevet: **09.09.2015**
(21) Numéro de dépôt: 11752299.5
(22) Date de dépôt: 26.07.2011
(51) Int. Cl.: C07K 14/46

(54) **NOUVEAUX PEPTIDES AVEC DES EFFETS ANALGESIQUES ET INHIBANT LES CANAUX ASIC**
NEUE PEPTIDE MIT SCHMERZSTILLENDER WIRKUNG ZUR HEMMUNG DES ASIC-KANALS
NOVEL PEPTIDES WHICH HAVE ANALGESIC EFFECTS AND WHICH INHIBIT ASIC CHANNELS

(30) Priorité: 26.07.2010 FR 1003136
(43) Date de publication de la demande: 05.06.2013
(73) Titulaire: Centre National de la Recherche Scientifique, 75794 Paris Cedex 16 (FR)
(72) Inventeur: LINGUEGLIA, Eric, F-06000 Nice (FR); DIOCHOT, Sylvie, F-06560 Valbonne (FR); BARON-FORSTER, Anne, F-06560 Valbonne (FR); SALINAS, Miguel, F-06800 Cagnes Sur Mer (FR); LAZDUNSKI, Michel, F-06000 Nice (FR)
(74) Mandataire: Novagraaf Technologies
(86) Numéro de dépôt international: PCT/FR2011/051800
(87) Numéro de publication internationale: WO 2012/022894

(56) Documents cités:
- HE YING-YING ET AL: "Cloning and purification of alpha-neurotoxins from king cobra (Ophiophagus hannah)", TOXICON, ELMSFORD, NY, US, vol. 44, no. 3, 1 septembre 2004 (2004-09-01), pages 295-303, XP009123146, ISSN: 0041-0101, DOI: DOI:10.1016/J.TOXICON.2004.06.003
- ESCOUBAS ET AL: "Isolation of a tarantula toxin specific for a class of proton-gated Na+ channels", JOURNAL OF BIOLOGICAL CHEMISTRY, AMERICAN SOCIETY FOR BIOCHEMISTRY AND MOLECULAR BIOLOGY, INC, US, vol. 275, no. 33, 18 août 2000 (2000-08-18), pages 25116-25121, XP002194541, ISSN: 0021-9258, DOI: DOI:10.1074/JBC.M003643200 cité dans la demande

## Description

### Domaine technique

La présente invention se rapporte à de nouveaux peptides qui induisent une analgésie et qui inhibent les canaux ASIC (Acid Sensing Ion Channel), plus particulièrement les canaux homomériques ASIC1a, homomériques ASIC1b et/ou hétéromériques contenant au moins une sous-unité choisie parmi ASIC1a et ASIC1b, aux polynucléotides codant pour lesdits peptides, ainsi qu'aux compositions pharmaceutiques, cellules hôtes et vecteurs les comprenant. En particulier lesdits peptides présentent au minimum 56% d'identité avec la séquence en acides aminés SEQ ID NO : 1 dans la liste de séquences annexée. Il s'agit notamment des deux peptides de 57 acides aminés, ASICalgine-1 (π-Dp1) et ASICalgine-2 (π-Dp2) isolés du venin de serpent *Dendroaspis polylepis* (mamba noir) de séquences SEQ ID NO : 2 et SEQ ID NO : 3, respectivement.

La présente invention se rapporte également à leur utilisation pour l'obtention d'un outil de diagnostic ou d'un médicament, en particulier d'un analgésique, ainsi que pour l'identification de molécules analgésiques ou inhibant les canaux ASIC.

La présente invention trouve notamment une application dans la prévention ou le traitement de la douleur, notamment celle associée à l'activation des canaux ASIC (par exemple les douleurs inflammatoires, neuropathiques, cancéreuses, post-opératoires, musculo-squelettiques, viscérales, etc.), des maladies neurologiques centrales (par exemple le stress post-traumatique, la dépression, l'anxiété, les accidents vasculaires cérébraux, l'épilepsie, la sclérose en plaques, les inflammations cérébrales, les maladies neurodégénératives, etc.), et des pathologies dans lesquelles l'implication des canaux ASIC a été proposée (par exemple les inflammations, les cancers, les fibromyalgies, le syndrome du colon irritable, etc.).

Dans la description ci-dessous, les références entre crochets **([ ])** renvoient à la liste des références présentées à la fin du texte.

### Etat de la technique

La prise en compte et le traitement de la douleur sont des aspects essentiels de l'amélioration de la qualité de vie des patients. La douleur touche un nombre considérable de personnes, de l'ordre de 60 millions en Europe chaque année, ce qui représente un coût annuel de 1 milliard de dollars en médicaments analgésiques pour son traitement. Les dépenses effectuées annuellement dans le monde en médicaments analgésiques peuvent être évaluées à environ 25 milliards de dollars, et devraient atteindre 42 milliards en 2010. La douleur se divise en deux catégories : la douleur aiguë et la douleur chronique. La douleur aiguë correspond à une douleur rapide et brève, limitée dans le temps. A l'opposé, la douleur chronique est une douleur persistante qui peut être liée, par exemple à une hyperalgésie, et qui constitue un énorme fardeau de la maladie, touchant environ 20% des adultes et 50% de la population la plus âgée.

Le traitement de la douleur repose essentiellement sur la prescription d'anti-inflammatoires, qu'ils soient non stéroïdiens (AINS) ou stéroïdiens (corticoïdes), et d'opiacés faibles ou forts. Les AINS forment la classe thérapeutique la plus prescrite au monde, du fait de leur grande efficacité tant sur l'inflammation que sur la douleur elle-même. Ils sont utilisés dans tous les types de douleurs inflammatoires qu'elles soient aiguës ou chroniques [Bertin et Vergne-Salle, 2007] [1]. Lorsque les AINS et/ou les corticoïdes ne suffisent pas à soulager une douleur inflammatoire, le prescripteur associe un antalgique non anti-inflammatoire, par exemple du paracétamol, des opioïdes faibles (codéine, tramadol), et si la douleur résiste encore au traitement, des opioïdes forts (morphine, oxycodone, fentanyl) [Gutstein & Akil, 2006] [2].

Si les AINS sont très efficaces, ils n'en restent pas moins de grands pourvoyeurs d'effets secondaires indésirables. Parmi les effets secondaires indésirables les plus classiques, les effets digestifs sont très fréquents et limitent l'utilisation des AINS dans de nombreuses situations cliniques. Il existe également des effets secondaires indésirables rénaux, cutanés, muqueux, allergiques et respiratoires, hématologiques, hépatiques et enfin neurosensoriels et psychiques [Bertin et Vergne-Salle, 2007, précité] [1]. En outre, les AINS ne sont pas efficaces dans tous les types de douleur. Les opioïdes jouent aussi un rôle majeur dans la lutte contre la douleur mais peuvent provoquer des phénomènes hallucinatoires et des dépressions cardio-respiratoires. Les analgésiques peuvent aussi être source de dépendance, comme par exemple la dépendance à la morphine, la méthadone, etc... Il existe aussi des cas d'accoutumance aux analgésiques, c'est-à-dire que la dose nécessaire pour obtenir un effet constant doit être augmentée. Cette accoutumance croit dans le temps et entraîne donc la nécessité d'augmenter les doses et peut entraîner l'inefficacité du médicament. En effet, la dose nécessaire pour soulager la douleur peut devenir supérieure à la dose toxique dudit médicament. Enfin le traitement par des opioïdes peut aussi être associé à des effets indésirables comme une constipation sévère ou une hyperalgésie à l'arrêt du traitement (douleur post-opératoire par exemple) [Gutstein & Akil, 2006, précité ; Bannister & Dickenson, 2010] [2, 3].

Malgré la diversité de l'arsenal thérapeutique existant, de nombreuses douleurs restent peu sensibles aux analgésiques connus, comme les douleurs neuropathiques consécutives à une atteinte du système nerveux (50% des patients ne sont pas soulagés), les douleurs viscérales chroniques telles que le syndrome du colon irritable ou les maladies inflammatoires chroniques de l'intestin, la fibromyalgie, les douleurs liées au cancers et métastases osseuses, etc. [Yennurajalingam et al., 2004; Mizoguchi et al., 2009] [4, 5].

Dans ce contexte, la découverte de nouveaux analgésiques ou antalgiques palliant ces défauts et inconvénients et/ou de nouvelles cibles analgésiques devrait donc représenter un réel progrès.

Les recherches de l'industrie pharmaceutique sur la douleur n'ont conduit ces dernières années qu'à quelques développements limités. On peut citer par exemple les tryptans pour la migraine et certains nouveaux médicaments dont l'usage reste encore limité comme l'association de tétrahydrocannabinol et cannabidiol pour les douleurs cancéreuses et neuropathiques. En fait, les progrès réalisés au cours des deux dernières décennies proviennent essentiellement d'une meilleure utilisation et d'ajustements de dosage des antalgiques disponibles. Aucune des grandes familles de ces antalgiques n'a un rapport bénéfice/risque qui soit optimal, à cause d'une efficacité limitée et/ou d'effets secondaires importants.

Parmi les cibles moléculaires identifiées ces dernières années, les canaux ioniques tiennent une place particulièrement importante car ils sont directement impliqués dans la détection et la transmission des signaux douloureux par les neurones sensoriels et centraux. Les canaux ASIC (Acid Sensing Ion Channels) sont des canaux cationiques activés par des acidifications du milieu extracellulaire (acidose extracellulaire) [Waldmann & Lazdunski, 1998; Wemmie et al., 2006 ; Lingueglia et al., 2007] [6, 7, 8]. Jusqu'à présent, quatre gènes codant pour au moins sept sous-unités (ASIC1a, ASIC1b, ASIC1b2, ASIC2a, ASIC2b, ASIC3 et ASIC4) ont été identifiés chez les mammifères. Des canaux ASIC fonctionnels résultent de l'association de différentes sous-unités ASIC en trimères [Jasti et al., 2007] [9] conduisant à des canaux homomères ou hétéromères [Lingueglia et al., 1997; Benson et al., 2002; Hesselager et al., 2004] [10, 11, 12]. Les canaux ASIC sont essentiellement exprimés dans les neurones sensoriels nocicepteurs du système nerveux périphérique et dans les neurones du système nerveux central [Waldmann et al., 1997a; Lingueglia et al., 2007, précité ; Noel et al., 2010] [13, 8, 14]. Alors que les isoformes ASIC1a et ASIC2 sont présents à la fois dans les systèmes nerveux central et périphérique, l'expression des isoformes ASIC1b et ASIC3 est restreinte aux neurones sensoriels [Waldmann et al., 1997b ; Bassler et al., 2001 ; Chen et al., 1998] [15, 16, 17].

Il a été postulé que les canaux ASIC exprimés par les neurones sensoriels, et notamment le canal ASIC3, sont capables de détecter les acidifications extracellulaires susceptibles de se développer lors d'une ischémie, d'une inflammation, d'un hématome, d'une fracture, d'une lésion, d'une intervention chirurgicale (douleur post-opératoire), ou du développement de certaines tumeurs [Reeh et Steen, 1996] [18]. Or il est connu depuis plusieurs années maintenant que l'acidose extracellulaire génère de la douleur [Steen et al., 1995a ; Issberner et al., 1996] [19, 20], et des expérimentations réalisées sur des volontaires humains sains [Ugawa et al., 2002 ; Jones et al., 2004] [21, 22] ont montré l'implication des canaux ASIC dans la douleur cutanée acide à l'aide de l'amiloride et de certains AINS, des inhibiteurs non spécifiques des canaux ASIC [Waldmann et al., 1997a, précité; Voilley et al., 2001] [13, 23]. Il a été également démontré le rôle important de certains canaux ASIC exprimés dans les neurones du système nerveux central dans l'activité neuronale (plasticité synaptique de l'hippocampe, de l'amygdale) et la neuromodulation de la transmission de l'information douloureuse (canaux ASIC1a) par les neurones de la moelle épinière [Noel et al., 2010] [14].

Jusqu'à récemment, le répertoire des ligands actifs capables d'inhiber les canaux ASIC était principalement limité à l'amiloride, à certains AINS et au composé A-317567 [Dubé et al., 2005] [24]. Cependant aucune de ces molécules n'est absolument spécifique des canaux ASIC ou d'une sous-unité ASIC particulière. Dans le but d'identifier des effecteurs spécifiques des canaux ASIC, un très grand nombre de venins de scorpion, d'abeille, d'araignée, de serpent ou d'anémone de mer a été criblé. Récemment deux toxines peptidiques animales, PcTx1 et APETx2, ont été identifiées qui inhibent les canaux ASIC1a homomériques et les canaux contenant la sous-unité ASIC3, respectivement [Escoubas et al., 2000 ; Diochot et al., 2004] [25, 26]. L'injection périphérique (sous-cutanée) d'APETx2 induit un effet analgésique sur la douleur inflammatoire et acide chez le rat [Deval et al., 2008] [27] et sur la douleur post-opératoire chez le rat après application intra-opératoire d'APETx2 [Deval et al., J. Neurosci., 31 (16) : 6059-6066, 2011] [36], alors que l'injection centrale de PcTx1 induit un effet analgésique puissant chez la souris [Mazzuca et al., 2007] [28]. Les effets analgésiques de ces ceux toxines ont permis de démontrer l'implication des canaux ASIC dans la perception et la transmission de l'information douloureuse.

Il existe donc un réel besoin d'identifier d'autres effecteurs spécifiques des canaux ASIC ou d'une sous-unité ASIC particulière, susceptibles de présenter un effet analgésique tout en palliant les défauts, inconvénients et obstacles des analgésiques de l'art antérieur.

### Description de l'invention

Les Inventeurs ont maintenant découvert et identifié à partir du venin du serpent *Dendroaspis polylepis* (mamba noir), de nouveaux peptides qui induisent une analgésie et qui inhibent les canaux ASIC (Acid Sensing Ion Channels), plus précisément les canaux homomériques ASIC1a, homomériques ASIC1b, hétéromériques ASIC1a+2a et hétéromériques ASIC1a+1b.

Il s'agit en particulier des peptides ASICalgine-1 (π-Dp1) et ASICalgine-2 (π-Dp2) qui, après injection centrale (intrathécale et intracérébroventriculaire) *in vivo* chez la souris, présentent un effet analgésique puissant sur des modalités douloureuses différentes (chimique, thermique, inflammatoire), qui est comparable à celui de la morphine mais largement indépendant de l'activation des récepteurs aux opiacés. En outre, ces peptides ne présentent pas d'effets neurotoxiques lorsqu'ils sont injectés par voie centrale. Par injection périphérique sous-cutanée, ces peptides exercent également un effet analgésique avec réversion d'une hyperalgésie inflammatoire. Ces deux peptides sont les premiers peptides extraits du venin de *Dendroaspis polylepis* (mamba noir) à présenter un effet analgésique.

Sans se trouver limité par cette explication, le mécanisme d'action le plus probable de ces peptides résulterait de l'inhibition des canaux ioniques ASIC qui jouent un rôle important dans la perception, la transmission et la modulation de l'information douloureuse. En effet ces peptides inhibent avec efficacité les canaux homomériques ASIC1a, homomériques ASIC1b et/ou hétéromériques contenant une sous-unité ASIC1a et/ou ASIC1b de rat et humains. Ces deux peptides sont notamment les premiers inhibiteurs connus des canaux homomériques ASIC1b et ASIC1a, et hétéromériques ASIC1a+ASIC1b et ASIC1a+ASIC2a.

Les peptides ASICalgine-1 (π-Dp1) et ASICalgine-2 (π-Dp2) inhibent également les courants ASIC natifs présents dans les neurones sensoriels et dans les neurones centraux. Ils n'ont pas d'effet sur le courant TRPV1 activé par la capsaïcine et par la chaleur et lui aussi impliqué dans la perception de la douleur. Ils ne modifient pas les propriétés électriques des neurones à l'état basal mais réduisent l'excitabilité neuronale en réponse à une acidification extracellulaire capable d'activer les canaux ASIC.

Les peptides ASICalgine-1 (π-Dp1) et ASICalgine-2 (π-Dp2) possèdent donc chez le rongeur des propriétés analgésiques de puissance comparable à celles de la morphine, qui continuent à être observées lorsque les récepteurs aux opiacés sont bloqués, et qui pourraient être expliquées par leur capacité à inhiber spécifiquement certains canaux ASIC de rongeurs et humains. Leur injection par voie centrale n'induit aucune toxicité (neurotoxicité, convulsions...), ni aucun effet sur l'activité motrice (test du rotarod accéléré).

Les peptides ASICalgine-1 (π-Dp1) et ASICalgine-2 (π-Dp2) peuvent donc être envisagés comme de nouvelles molécules ayant un potentiel thérapeutique contre la douleur (par exemple la douleur cancéreuse, neuropathique, post-opératoire, etc...) chez l'homme.

La présente invention a donc pour objet un peptide comprenant :
(i) la séquence en acides aminés où X⁴ représente n'importe quel acide aminé ;
   ou
(ii) une séquence naturelle ou synthétique présentant une identité d'au moins 56% avec la séquence SEQ ID NO: 1, de préférence d'au moins 70%, préférentiellement d'au moins 80%, tout préférentiellement d'au moins 98%, et conservant les propriétés biologiques des peptides comprenant la séquence SEQ ID NO : 1 telles que décrites ci-dessus, à savoir qui induisent une analgésie et qui inhibent au moins un canal ASIC, en particulier au moins un canal ASIC contenant au moins une sous-unité choisie dans le groupe constitué des sous-unités ASIC1a et ASIC1b.

Lesdits peptides selon l'invention fonctionnent plus particulièrement en tant que bloqueur des canaux homomériques ASIC1a, homomériques ASIC1b et/ou des canaux ASIC hétéromériques contenant au moins une sous-unité choisie dans le groupe constitué des sous-unités ASIC1a et ASIC1b, en particulier des canaux hétéromériques ASIC1a+ASIC1b et/ou hétéromériques ASIC1 a+ASIC2a.

On entend par « bloqueur » au sens de la présente invention, un peptide capable d'inhiber de façon concentration-dépendante le courant produit par les canaux susnommés. Par exemple, il s'agit d'un peptide qui comme la PcTx1 [Escoubas et al., 2000, précité] [25] est capable d'inhiber à une concentration de 1 nM, 50% du courant produit par les canaux ASIC1a homomériques, ou qui, comme APETx2 [Diochot et al., 2004, précité] [26], est capable d'inhiber à une concentration de 2 µM, 50% du courant produit par les canaux ASIC1a+ASIC3 hétéromériques de rat.

De préférence lesdits peptides selon l'invention sont extraits du venin du serpent *Dendroaspis polylepis.* Par exemple, ils sont isolés du venin par fractionnement séquentiel à l'aide d'une chromatographie liquide à haute performance à polarité de phase inversée (RP-HPLC). Lesdits peptides peuvent également être préparés par des procédés de recombinaison ADN ou par synthèse chimique.

De préférence, lesdits peptides selon l'invention comprennent le peptide ASICalgine-1 (π-Dp1) ou ASICalgine-2 (π-Dp2) présentant la séquence SEQ ID NO : 1 où X⁴ représente Y ou F, respectivement (SEQ ID NO : 2 et 3, respectivement).

La présente invention a encore pour objet un polynucléotide comprenant une séquence en nucléotides codant pour un peptide selon l'invention.

De préférence ledit polynucléotide selon l'invention comprend une séquence en nucléotides : ou où ⁷³txx⁷⁵ et ¹⁰txx¹² représente tac, tat ou ttt, ttc.

De préférence ledit polynucléotide selon l'invention comprend une séquence en nucléotides telle qu'il peut s'hybrider en conditions stringentes avec la séquence en nucléotides SEQ ID NO : 4 ou SEQ ID NO : 22, ou une séquence complémentaire de celle-ci. Par exemple, il s'agit d'un polynucléotide qui comprend une séquence naturelle ou synthétique présentant une identité d'au moins 76% avec la séquence SEQ ID NO: 4 ou la séquence SEQ ID NO : 22, de préférence d'au moins 80%, tout préférentiellement d'au moins 98%.

La présente invention a encore pour objet un vecteur comprenant un polynucléotide selon l'invention.

De préférence ledit vecteur selon l'invention est un vecteur d'expression.

La présente invention a encore pour objet une cellule hôte comprenant un vecteur selon l'invention.

La présente invention a encore pour objet une composition pharmaceutique comprenant un ou plusieurs peptide selon l'invention, polynucléotide selon l'invention, vecteur selon l'invention, ou cellule hôte selon l'invention. Une composition pharmaceutique selon l'invention peut également comprendre un ou plusieurs véhicule pharmaceutiquement acceptable (carbonate de calcium, amidon, talc lactose, stéarate de magnésium, gomme d'acacia, etc.), et se présenter sous forme de solution, suspension, pâte, gélule, comprimé, capsule, poudre, granule, lyophilisé, système à libération contrôlée, microparticule, micro- ou nanosphère, liposome, etc.. Une composition pharmaceutique selon l'invention peut être administrée par voie orale, intramusculaire, intraveineuse, sous-cutanée, topique, pulmonaire, intranasale, buccale, rectale, sublingale, intradermique, intrapéritonéale, intrathécale, etc.. La quantité efficace de principe actif (peptide, polynucléotide, vecteur, ou cellule hôte selon l'invention), dans une composition pharmaceutique selon l'invention, peut varier de sorte qu'une dose efficace soit obtenue et qu'une quantité analgésique soit administrée à un mammifère. Le dosage administré à un mammifère particulier dépend de plusieurs facteurs : la voie d'administration, la durée du traitement, la taille et la condition physique du mammifère, la puissance du principe actif et la réponse du mammifère audit principe actif. Par exemple, une quantité analgésique efficace de principe actif administré par voie intrathécale va généralement d'environ 5 ng/kg à 500 µg/kg de poids corporel du mammifère, de préférence d'environ 50 ng/kg à 50 µg/kg de poids corporel du mammifère, tout préférentiellement d'environ 500 ng/kg à 5 µg/kg de poids corporel du mammifère. Les quantités efficaces de principe actif peuvent varier lorsque d'autres voies d'administration sont utilisées. Une quantité analgésique efficace peut être estimée en testant le principe actif dans un ou plusieurs test de douleur cité ci-après à une dose pouvant varier selon un ou plusieurs critères cité ci-dessus afin de déterminer la quantité efficace de principe actif à administrer à un mammifère.

La présente invention a encore pour objet une substance choisie parmi un peptide selon l'invention, un polynucléotide selon l'invention, un vecteur selon l'invention, une cellule hôte selon l'invention, ou une composition pharmaceutique selon l'invention, pour son application comme médicament.

De préférence ledit médicament est un analgésique, par exemple destiné à la prévention ou au traitement d'une douleur impliquant l'activation des canaux ASIC, en particulier des canaux ASIC contenant au moins une sous-unité choisie dans le groupe constitué des sous-unités ASIC1a et ASIC1b, tout particulièrement des canaux homomériques ASIC1a, homomériques ASIC1b, hétéromériques ASIC1a+ASIC1b et/ou hétéromériques ASIC1a+ASIC2a. Par exemple, la douleur peut (i) résulter de l'activation des canaux ASIC centraux ou périphériques ou (ii) induire leur activation. Par exemple, il s'agit de douleurs, notamment celles associées à l'activation des canaux ASIC, choisies dans le groupe comprenant les douleurs inflammatoires, neuropathiques, cancéreuses, post-opératoires, musculo-squelettiques, viscérales, etc..

De préférence ledit médicament est destiné à la prévention ou au traitement d'une pathologie impliquant l'activation des canaux ASIC, en particulier des canaux ASIC contenant au moins une sous-unité choisie dans le groupe constitué des sous-unités ASIC1a et ASIC1b, tout particulièrement des canaux homomériques ASIC1a, homomériques ASIC1b, hétéromériques ASIC1 a+ASIC1 b et/ou hétéromériques ASIC1a+ASIC2a. Par exemple, il s'agit des pathologies choisies dans le groupe comprenant les inflammations, les cancers, les fibromyalgies, le syndrome du colon irritable, etc...

De préférence ledit médicament est destiné à la prévention ou au traitement d'une maladie neurologique centrale, par exemple choisie dans le groupe comprenant le stress post-traumatique, la dépression, l'anxiété, les accidents vasculaires cérébraux, l'épilepsie, les inflammations centrales, la sclérose en plaque, les maladies neurodégénératives, etc...

De préférence ledit médicament est administré par voie parentérale, à savoir loco-régionale ou centrale (intrapéritonéale, péridurale, intrathécale, intracérébroventriculaire, intradermique, etc.) et générale (intramusculaire, intraveineuse, sous-cutanée, etc...), voie orale, voie locale (trans-cutanée, etc...) ou voie respiratoire (inhalation, instillation, etc...). Tout préférentiellement, ledit médicament est administré par voie intrathécale, péridurale, intracérébroventriculaire, intrapéritonéale ou sous-cutanée.

La présente invention a encore pour objet une substance choisie parmi un peptide selon l'invention, un polynucléotide selon l'invention, un vecteur selon l'invention, une cellule hôte selon l'invention, ou une composition pharmaceutique selon l'invention, pour son application comme outil diagnostic.

La présente invention a encore pour objet un procédé d'identification d'un composé mimant l'activité analgésique d'un peptide selon l'invention, comprenant les étapes suivantes :
a) déterminer l'activité analgésique d'un peptide selon l'invention ;
b) déterminer l'activité analgésique d'un composé candidat ;
c) comparer les activités analgésiques obtenues aux étapes a) et b) ;
d) sélectionner le composé candidat présentant une activité analgésique équivalente ou supérieure à celle d'un peptide selon l'invention.

La présente invention a encore pour objet un procédé d'identification d'un composé mimant l'activité analgésique d'un peptide selon l'invention, comprenant les étapes suivantes :
a) mettre en contact un peptide selon l'invention avec un échantillon, et mesurer la liaison dudit peptide avec ledit échantillon ;
b) ajouter un composé candidat, et évaluer l'effet dudit composé sur la liaison dudit peptide avec ledit échantillon ;
c) sélectionner le composé candidat capable de moduler la liaison dudit peptide avec ledit échantillon.

On entend par « composé mimant l'activité analgésique d'un peptide selon l'invention » au sens de la présente invention, un composé candidat capable d'induire une analgésie et de lier les canaux ASIC auxquels les peptides selon l'invention se lient ou encore d'agir d'une manière physiologique identique ou similaire à celle des peptides selon l'invention, à savoir de moduler (inhiber ou stimuler) de façon réversible et concentration-dépendante le courant produit par au moins un canal contenant au moins une sous-unité choisie dans le groupe constitué des sous-unités ASIC1a et ASIC1b, en particulier par des canaux homomériques ASIC1a, homomériques ASIC1b, hétéromériques ASIC1a+ASIC1b et/ou hétéromériques ASIC1a+ASIC2a. Par exemple, il s'agit d'un peptide qui comme la PcTx1 [Escoubas et al., 2000, précité] [25] est capable d'inhiber à une concentration de 1 nM, 50% du courant produit par les canaux ASIC1a homomériques, ou qui, comme APETx2 [Diochot et al., 2004, précité] [26], est capable d'inhiber à une concentration de 2 µM, 50% du courant produit par les canaux hétéromériques ASIC1 a+ASIC3 de rat.

On entend par « échantillon » au sens de la présente invention, des cellules ou tissu préalablement isolés à partir de l'organisme entier ou de lignées cellulaires immortalisées, d'insectes ou de mammifères.

Par « activité analgésique », il est fait référence à la capacité d'un peptide selon l'invention à traiter la douleur chez un mammifère, ou d'atténuer la douleur comme mis en évidence par un ou plusieurs modèle de laboratoire conventionnel pour tester la douleur ou évaluer l'analgésie, tels que ceux décrits ci-après. Ainsi, l'activité analgésique d'un peptide selon l'invention peut être déterminée par exemple à l'aide d'un ou plusieurs des tests *in vivo* suivants : i) latence du retrait de la queue/patte (mesure de la nociception thermique) [Abott et al., 1982 ; Cridland et Henry, 1992] [29, 30], ii) seuil sur plaque chaude/froide (mesure de la nociception thermique) [Woolfe et Macdonald, 1944; Ankier, 1974] [31, 32], iii) seuil sur filament vonFrey ou test de Randall-Selitto ou test de la pince instrumentée (mesure de l'activité nociceptive mécanique) [Kim et al., 1993] [33], iv) test de distribution pondérale dynamique (mesure de l'activité nociceptive liée à la posture), v) mesure des comportements nociceptifs spontanés et/ou d'un ou plusieurs tests *in vitro* : par exemple le criblage de composés candidats par compétition où un peptide selon l'invention marqué (par exemple avec un radiomarqueur tel que C¹⁴, H³, I¹²⁵, une enzyme telle que la péroxydase, la phosphatase alcaline ou acide, un marqueur fluorescent tel que FITC, rhodamine, un anticorps, un antigène, biotine, ion paramagnétique, une particule de latex, etc...) est mis en contact avec un échantillon dans des conditions permettant sa liaison audit échantillon, la liaison dudit peptide marqué lié à l'échantillon est mesurée, et où les composés mimant l'activité dudit peptide marqué entrent en compétition avec ledit peptide pour la liaison aux sites sur le récepteur (canaux ASIC contenant au moins une sous-unité choisie dans le groupe constitué des sous-unités ASIC1a et ASIC1b). Ainsi une quantité plus faible de marquage détectable est mesurée lorsque les composés test miment l'activité dudit peptide en se liant au récepteur que lorsque les composés test ne miment pas l'activité dudit peptide et ne lient pas le récepteur, ou se lient avec une moindre affinité. En alternative, le criblage par compétition peut être réalisé avec le marquage du composé candidat au lieu de celui du peptide selon l'invention. Ainsi une quantité plus élevée de marquage détectable sera mesurée lorsque les composés test miment l'activité dudit peptide en se liant au récepteur que lorsque les composés test ne miment pas l'activité dudit peptide et ne lient pas le récepteur, ou se lient avec une moindre affinité.

Des exemples de procédés d'identification de composés mimant l'activité analgésique de peptides sont décrits (sans se trouver limité par cette description), par exemple dans le Brevet US 5,877,026 [34].

D'autres avantages pourront encore apparaître à l'homme du métier à la lecture des exemples ci-dessous, illustrés par les figures annexées, donnés à titre illustratif.

### Brève description des figures

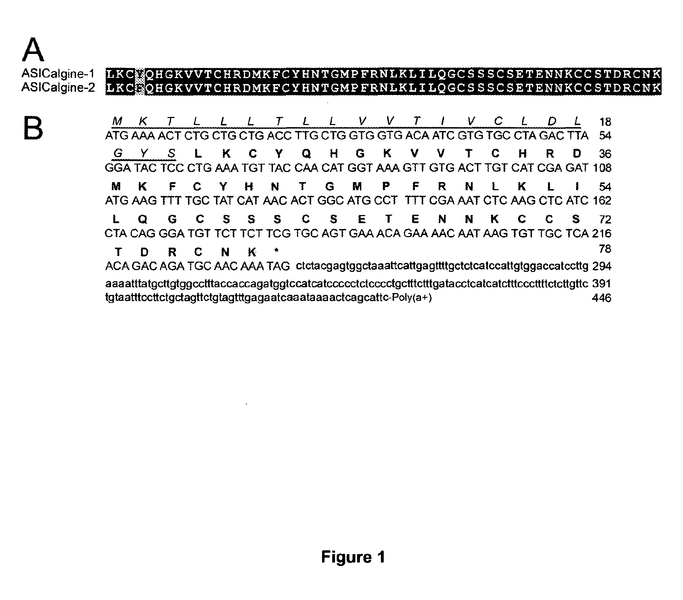

- La figure 1 représente (A) la séquence en acides aminés des peptides ASICalgine-1 (π-Dp1) et ASICalgine-2 (π-Dp2) isolés du venin du serpent *Dendroaspis polylepis* (SEQ ID NO : 2 et 3, respectivement) (B) la séquence de nucléotides de l'ADN complémentaire (ADNc) codant pour ASICalgine-1 (SEQ ID NO: 5). La séquence signal (en italique) et le peptide final (en gras) (cf. SEQ ID NO : 6), le codon stop (*) sont indiqués. La séquence de l'extrémité 5' non codante n'est pas représentée (correspondant à la SEQ ID NO : 9).

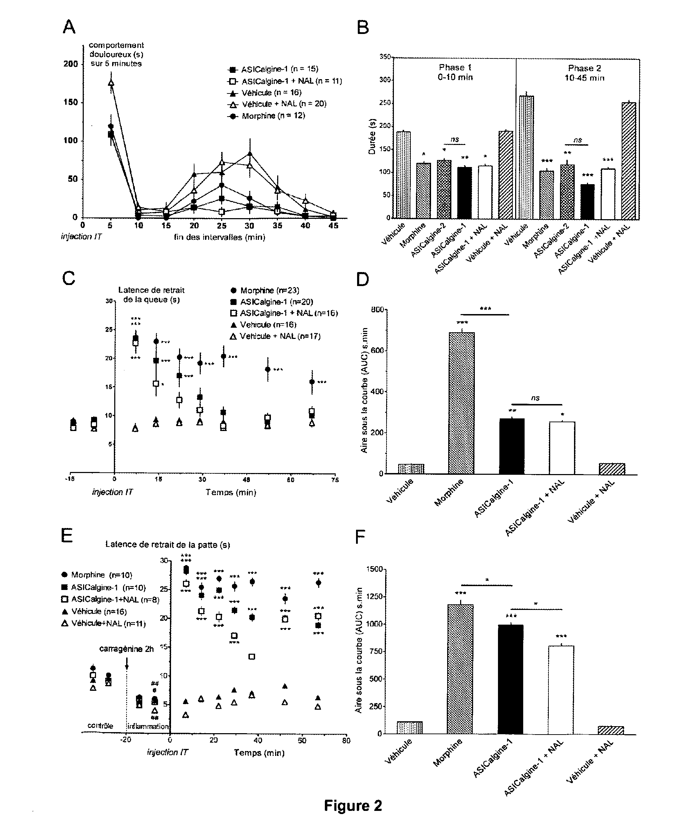

- La figure 2 représente l'effet analgésique des peptides ASICalgine-1 (π-Dp1) et ASICalgine-2 (π-Dp2) *in vivo* après injection intrathécale chez la souris. Douleur chimique (formol) : (A) cinétique du comportement douloureux spontané mesuré par intervalle de 5 minutes (B) Durée totale de la phase I (0-10 minutes) de douleur aiguë et de la phase Il (10-45 minutes) de douleur inflammatoire. Douleur aiguë thermique : (C) cinétique de la latence de retrait de la queue (D) moyenne des aires sous la courbe (AUC) mesurées pour chaque animal. Hyperalgésie inflammatoire : (E) cinétique de la latence de retrait de la patte. Significativité de l'hyperalgésie par rapport à la valeur contrôle: #, p<0,05 ; ##, p<0,01 ; ###, p<0,001 ; par test ANOVA suivi d'une comparaison multiple de Newman Keuls. (F) moyenne des aires sous la courbe (AUC) mesurées pour chaque animal à partir du niveau hyperalgésié (T-7 min). Les valeurs moyennes ± sem (erreur standard à la moyenne) sont figurées et le nombre (n) d'animaux testés est mentionné dans les légendes. Significativité par rapport à l'injection du véhicule ± naxolone (NAL) ou bien selon indiqué sur la figure par un trait: non significatif *ns*, p>0,05; *, p<0,05; **, p<0,01 ; ***, p<0,001 ; par test ANOVA suivi d'une comparaison multiple de Newman Keuls.

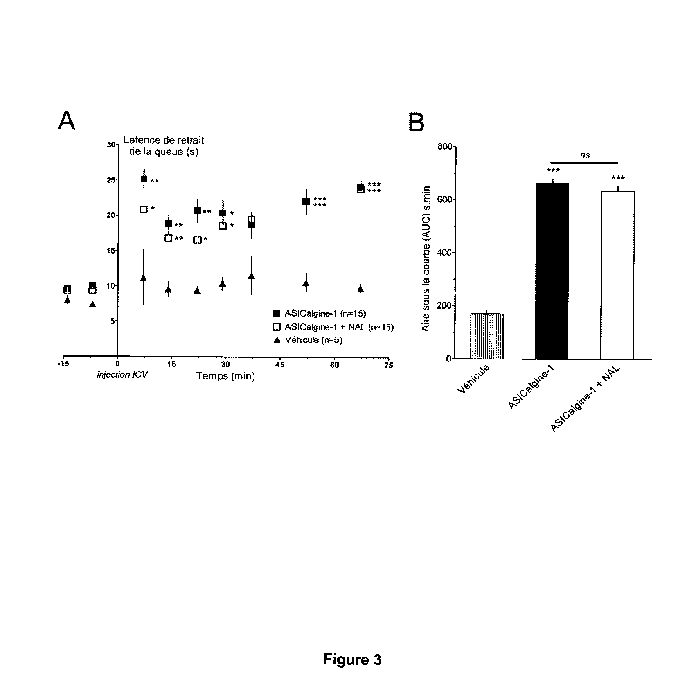

- La figure 3 représente l'effet analgésique du peptide ASICalgine-1 (π-Dp1) *in vivo* après injection intracérébroventriculaire chez la souris dans le test douleur aiguë thermique : (A) cinétique de la latence de retrait de la queue (B) moyenne des aires sous la courbe (AUC) mesurées pour chaque animal. Les valeurs moyennes ± sem (erreur standard à la moyenne) sont figurées. Le nombre (n) d'animaux testés est mentionné dans les légendes. Significativité par rapport à l'injection du véhicule ou bien selon indiqué sur la figure par un trait : non significatif *ns*, p>0,05; *, p<0,05 ; **, p<0,01 ; ***, p<0,001 ; par ANOVA suivi d'une comparaison multiple de Newman Keuls.

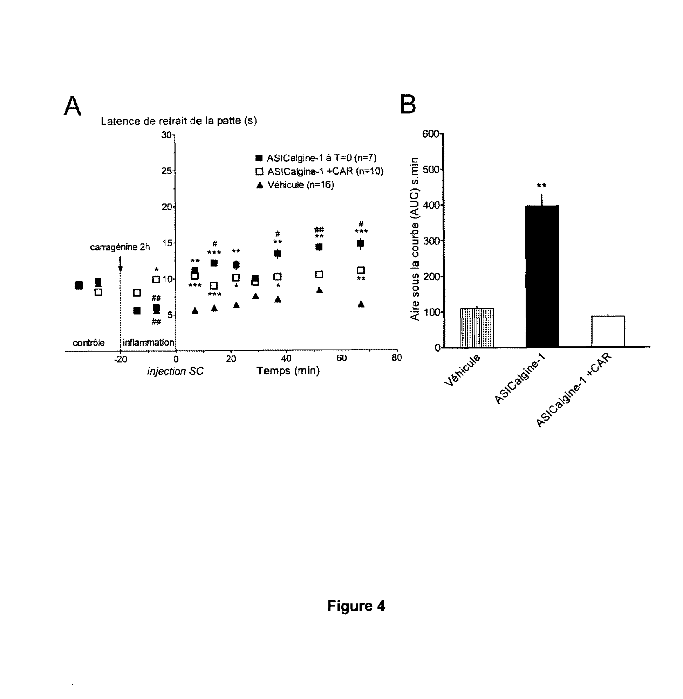

- La figure 4 représente l'effet analgésique du peptide ASICalgine-1 (π-Dp1) *in vivo* après injection sous-cutanée dans le test d'hyperalgésie inflammatoire : (A) cinétique de la latence de retrait de la patte. Le peptide ASICalgine-1 est injecté par voie sous-cutanée dans la patte arrière gauche en même temps que la carragénine (□) puis à nouveau deux heures après (temps 0 ou T0, □). Une procédure similaire est effectuée mais avec injection du peptide ASICalgine-1 uniquement à T0, soit après l'apparition de l'hyperalgésie inflammatoire (■).(B) moyenne des aires sous la courbe (AUC) estimées pour chaque animal à partir de la valeur à T-7 min. Les valeurs moyennes ± sem sont figurées. Le nombre (n) d'animaux testés est mentionné dans les légendes. Significativité par rapport à l'injection du véhicule: *, p<0,05 ; **, p<0,01 ; ***, p<0,001 ; par test ANOVA suivi d'une comparaison multiple de Newman Keuls. Significativité par rapport à la valeur contrôle avant l'injection de carragénine: #, p<0,05 ; ##, p<0,01 ; ###, p<0,001 ; par T-test apparié.

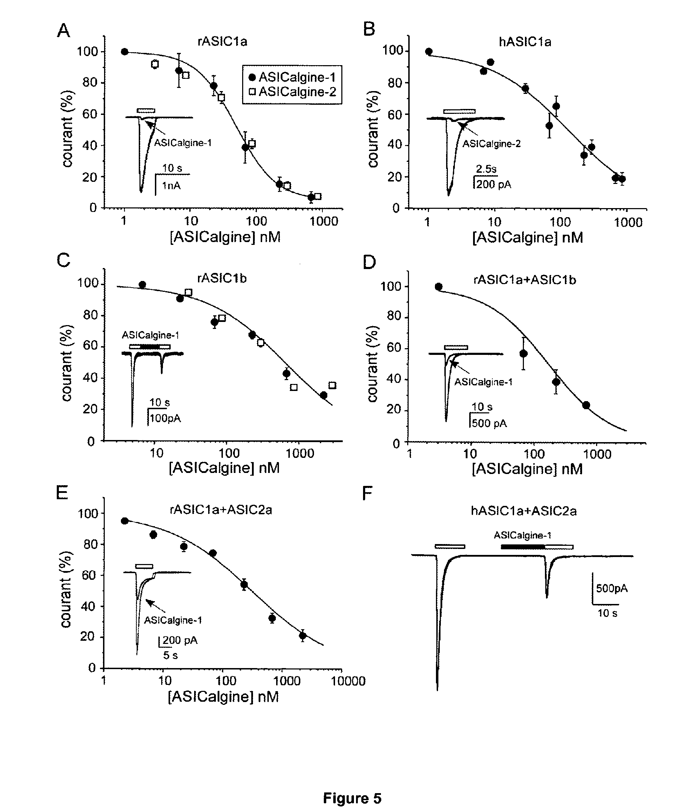

- La figure 5 représente l'inhibition par les peptides ASICalgine-1 (π-Dp1) et ASICalgine-2 (π-Dp2) des courants générés par les canaux ASIC exprimés de façon hétérologue dans les cellules COS. Courbe dose-réponse de l'inhibition par ASICalgine-1 (π-Dp1) du courant homomérique ASIC1a de rat, CI₅₀=49 nM (A), du courant homomérique ASIC1a humain, CI₅₀=127nM (B), du courant homomérique ASIC1b de rat, CI₅₀=650 nM (C), du courant hétéromérique ASIC1a+ASIC1b (ratio 1:1) de rat, CI₅₀=223 nM (D), du courant hétéromérique ASIC1a+ASICa (ratio 2 :1) de rat, CI₅₀=308 nM (E). Les valeurs moyennes ± sem sont indiquées, avec n de 4 à 15 expériences. Des traces originales de courants inhibés par ASICalgine-1 (π-Dp1, 674 nM) et ASICalgine-2 (π-Dp2, 852 nM) sont montrées en miniature. (F) Traces originales de courant montrant l'inhibition du courant hétéromérique ASIC1a+ASIC2a (ratio 2 :1) humain par ASICalgine-1 (π-Dp1, 674 nM). Potentiel de repos : -60 mV, barres blanches : saut de pH de 7,4 à 5,5, barres noires : application du peptide.

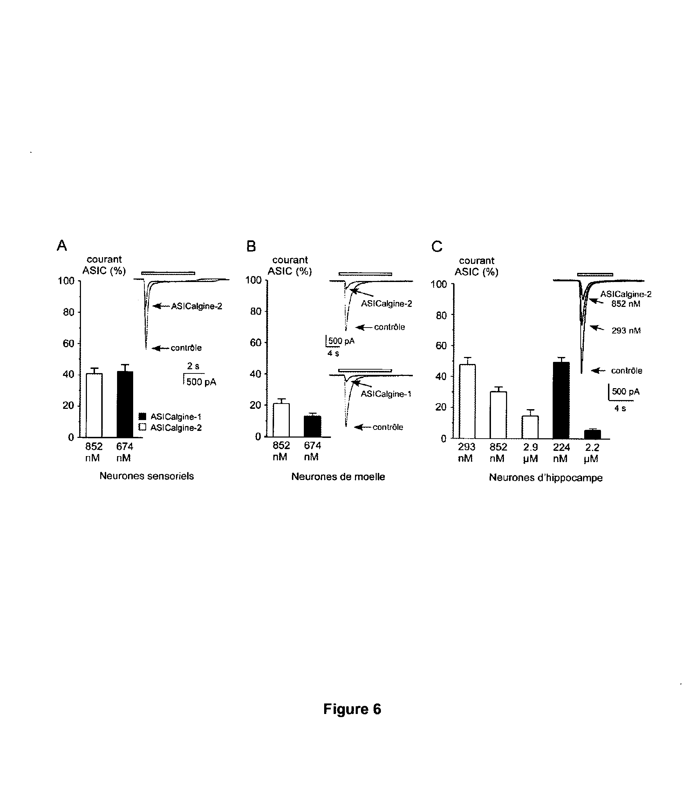

- La figure 6 représente l'inhibition par les peptides ASICalgine-1 (π-Dp1) et ASICalgine-2 (π-Dp2) des courants ASIC natifs des neurones sensoriels et centraux murins en culture. (A) Inhibition moyenne du courant ASIC des neurones sensoriels de rat (n=34) par ASICalgine-2 (̂π-Dp2, 852 nM, □) et ASICalgine-1 (π-Dp1, 674 nM, ■). A droite, exemples de courants originaux enregistrés au potentiel de repos de -50 mV et activés par un saut de pH de 7,4 à 6 (barre blanche). Dans les neurones sensoriels, le courant ASIC total résulte d'un mélange de courant ASIC1a homomérique, de courant de type ASIC1b et de courant de type ASIC3. (B) Inhibition moyenne du courant ASIC des neurones de moelle dorsale de souris (n=18) par ASICalgine-2 (π-Dp2, 852 nM, □) et ASICalgine-1 (π-Dp1, 674 nM, ■). A droite, exemple de courants originaux enregistrés au potentiel de repos de -50 mV et activés par un saut de pH de 7,4 à 6 (barre blanche). (C) Inhibition moyenne du courant ASIC des neurones d'hippocampe de souris (n=26) par ASICalgine-2 (π-Dp2, 293 nM, 852 nM et 2,9 µM, □) et ASICalgine-1 (π-Dp1, 224 nM et 2,2 µM, ■). A droite, exemple de courants originaux enregistrés au potentiel de repos de - 50 mV et activés par un saut de pH de 7,4 à 5,5 (barre blanche). Dans les neurones centraux (moelle et hippocampe de souris), le courant ASIC total résulte d'un mélange de courant ASIC1a homomérique et de courant ASIC1a+ASIC2a hétéromérique. Les valeurs moyennes ± sem sont indiquées.

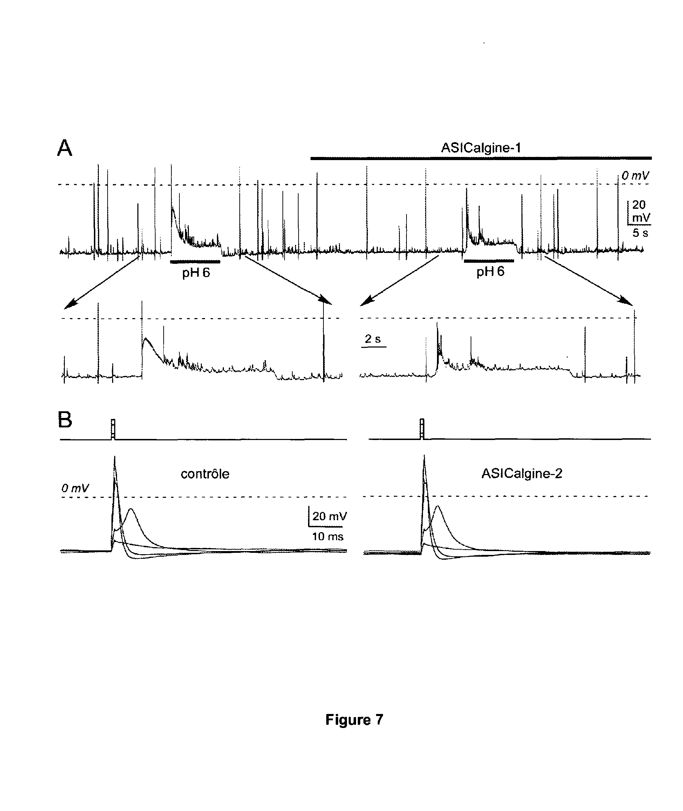

- La figure 7 représente l'effet des peptides ASICalgine-1 (π-Dp1) et ASICalgine-2 (π-Dp2) sur le potentiel de membrane de neurones de moelle épinière dorsale de souris. (A) Potentiel membranaire d'un neurone où les potentiels d'action spontanés traduisent une activité synaptique (ligne pointillée : 0 mV). L'activation du courant ASIC par un saut de pH de 7,4 à 6 induit une dépolarisation dont le pic déclenche un potentiel d'action (agrandissement). L'application du peptide ASICalgine-1 (π-Dp1, 674 nM) ne modifie pas le potentiel de repos du neurone ni l'activité spontanée. En revanche, la dépolarisation induite par le saut de pH acide est réduite et n'induit plus de potentiel d'action. (B) Potentiel d'action évoqué par une stimulation électrique (créneaux de courant figurés au dessus). Quatre réponses sont superposées (panneau de gauche) : deux dépolarisations infraliminaires et deux autres déclenchant un potentiel d'action (ligne pointillée : 0 mV). L'application d'ASICalgine-2 (π-Dp2, 852 nM, panneau de droite) ne modifie pas l'excitabilité du neurone.

### EXEMPLES

### EXEMPLE 1 : ISOLEMENT DE DEUX PEPTIDES, ASICALGINE-1 (π-Dp1) ET ASICALGINE-2 (π-Dp2), A PARTIR DU VENIN DU SERPENT DENDROASPIS POLYLEPIS

### Venin et purification des toxines peptidiques

Le venin lyophilisé provient de la société Latoxan (Valence, France). Le venin a été dilué dans l'acide acétique (1%) et fractionné par passage sur une colonne contenant une résine Sephadex G50. La fraction peptidique a été récupérée et ensuite fractionnée sur un système de Chromatographie Liquide Haute Pression (HPLC) : 1) La fraction peptidique a été chargée et éluée sur une colonne échangeuse de cations avec un gradient de pH avec les solvants (acide acétique 1% pH3,0 et acétate d'ammonium 1 M pH6,8). La fraction contenant les ASICalgines a été éluée à partir de 60% d'acétate d'ammonium. 2) Cette fraction peptidique a été chargée sur une colonne de phase inverse C18 et ses composants ont été élués par un gradient d'hydrophobicité entre les solvants eau-TFA 0,1% et Acétonitrile-TFA 0,1%. Les peptides ASICalgine-1 et ASICalgine-2 ont été élués purs à 26 et 27% d'acétonitrile respectivement.

### Caractérisation des peptides

### Analyse en acides aminés

ASICalgine-1 et ASICalgine-2 sont deux isopeptides basiques présentant une séquence de 57 acides aminés contenant 8 cystéines formant 4 ponts disulfures (Figure 1A, SEQ ID NO: 2 et 3, respectivement), et précédée d'une séquence signal de 21 acides aminés (Figure 1 B, cf. SEQ ID NO : 6). Ils ne diffèrent l'un de l'autre que par un acide aminé en position 4 de SEQ ID NO : 1, 2 ou 3 (= position 25 de SEQ ID NO : 6).

### Détermination de la séquence en acides aminés par clonage

La séquence complète (Figure 1B) d'ASICalgine-1 a été déterminée par clonage de l'ADNc par PCR, à partir des ARN messagers (ARNm) présents à l'état de traces dans le venin brut. 25 mg de venin de serpent *Dendroaspis polylepsis* (Sigma) ont été resuspendus dans du tampon de lyse : 500 mM LiCl, 10 mM EDTA, 1% (w/v) LiDS, et 5 mM dithiothréitol dans 100 mM de tampon Tris-HCl, pH 7,5. Les ARN messagers ont été capturés grâce à des billes magnétiques greffées d'oligonucléotides dT25 (Dynal, UK). Ces oligonucléotides ont été directement utilisés comme amorces pour réaliser la transcription inverse de l'ARNm en ADNc avec la transcriptase inverse PrimeScript^{™} (TaKaRa Bio Inc., Japan). La banque d'ADNc en phase solide ainsi obtenue a été utilisée pour réaliser une PCR dégénérée avec les oligonucléotides sens (TGITTYCARCAYGGIAARGT, SEQ ID NO : 7) et antisens (YTTIARRTTICGRAAIGGCAT, SEQ ID NO : 8) définis à partir de la séquence protéique partielle de la toxine déterminée par séquençage N-terminal direct. Pour exclure toute erreur due à la polymérase, trois PCR indépendantes ont été réalisées qui ont produit des fragments à la taille espérée (89 paires de bases), ensuite sous-clonés dans le vecteur pGEMTeasy (Promega) et séquencés. Grâce à ces séquences, un oligonucléotide-sens spécifique additionné d'un site de restriction EcoRI a été synthétisé (ACAC(GAATTC)GCTATCATAACACTGGCATG, SEQ ID NO: 9), ainsi qu'un oligonucléotide-antisens non-spécifique poly-dT30 également additionné d'un site EcoRI (ACAC(GAATTC)dT30, SEQ ID NO : 10). Ces deux oligonucléotides ont permis d'amplifier par PCR toute l'extrémité 3'-codante et 3' non-codante à fin d'identification. Trois PCR indépendantes ont généré une bande d'environ 400 paires de bases et son sous-clonage dans le vecteur pGEMTeasy au site de restriction ECoRI a permis son séquençage en excluant toute erreur générée par la polymérase.

A partir des séquences 3'- et 5'- non-codantes, un oligonucléotide-sens (ACAC(GAATTC)TCCAGAGAAGATCGCAAG***ATG***, SEQ ID NO : 11) et antisens (ACAC(GAATTC)ATTTAGCCACTCGTAGAG***CTA***, SEQ ID NO : 12) encadrant la séquence codante ont été synthétisés. Quatre PCR indépendantes ont été faites sur la banque de cDNA en phase solide et les produits de PCR ont été à nouveau sous-clonés dans le vecteur pGEMTeasy pour obtenir la séquence nucléotidique complète du précurseur de la toxine ASICalgine-1.

### Séquençage peptidique

La séquence a été confirmée jusqu'à l'acide aminé Asp40 par séquençage N-terminal direct des peptides réduits et alkylés sur séquenceur automatique (LC491, Applied Biosystems, USA).

### Clivage enzymatique

Pour confirmer les séquences, les peptides ont été soumis aux traitements enzymatiques suivants: (a) protéase V8 avec une séparation des peptides digérés sur une colonne HPLC de phase inverse C18. Le clivage en position D15 et E43 et la séparation sur HPLC de 2 peptides de masse moléculaire 1786.9 Da (peptide 1-15), entièrement re-séquencé, et 3194.5 Da (peptide 16-43), séquence partielle, permettent de confirmer la séquence N-terminale. (b) digestion à la trypsine avec analyse directe par spectrométrie de masse. Les fragments tryptiques confirment aussi la séquence N-terminale ainsi que d'autres parties de la séquence peptidique des ASICalgines. Les peptides tryptiques détectés et séquencés par analyse MS/MS sont présentés dans le tableau 1 ci-dessous

**Tableau 1**

| Position | Séquence peptidique | Mw (Da) |
|---|---|---|
| 3-8 | CFQHGK (SEQ ID NO: 13) | 775.27 |
| 3-8 | CYQHGK (SEQ ID NO: 14) | 791.27 |
| 3-14 | CFQHGKVVTCHR (SEQ ID NO: 15) | 1527.58 |
| 3-14 | CYQHGKVVTCHR (SEQ ID NO: 16) | ND |
| 9-14 | VVTCHR (SEQ ID NO: 17) | 770.31 |
| 15-28 | DMKFCYHNTGMPFR (SEQ ID NO: 18) | 1803.57 |
| 32-48 | LILQGCSSSCSETENNK (SEQ ID NO: 19) | 1925.63 |
| 49-54 | CCSTDR (SEQ ID NO: 20) | 797.24 |
| 32-54 | LILQGCSSSCSETENNKCCSTDR (SEQ ID NO: 21) | 2705.78 |

### Spectrométrie de masse (MALDI-TOF)

Dans tous les cas l'analyse de spectrométrie de masse a été réalisée sur un spectromètre 4800 MALDI-TOF-TOF (Applied Biosystems, USA).

Les protéines natives ont été analysées en mode linéaire ion positif avec une matrice d'acide sinapinique (Sigma Aldrich, USA) en utilisant une calibration interne avec un mélange de protéines de masse moléculaire connues.

La masse moléculaire mesurée est de 6554.8 Da pour ASICalgine-1, et de 6538.4 pour ASICalgine-2. Elle est en parfait accord avec les masses moléculaires calculées par la séquence 6554.58Da et 6538.58 Da, respectivement, sur le logiciel GPMAW protein analysis. Ces résultats suggèrent une extrémité C-terminale acide carboxylique libre. L'absorbance moléculaire calculée à 280 nm est de ε= 3040 (pour ASICalgine-1) et ε = 1760 (pour ASICalgine-2).

### Recherche d'homologues dans les bases de données

ASICalgine-1 et ASICalgine-2 ne figurent pas dans les bases de données et ne présentent aucune identité de séquence totale avec d'autres peptides ou toxines déjà connues.

Le programme d'alignement de séquence BLAST montre que ASICalgine-1 et ASICalgine-2 présentent néanmoins 47 à 55% d'identité de séquence (63 à 66% d'homologies de séquence) avec les peptides CM-1 b, CM-3 et CM-2a purifiés à partir de venins de diverses espèces de serpents Cobra (Naja et Ringhals), ainsi qu'avec une neurotoxine-α OH-26 présente dans le venin du cobra royal *Ophiophagus hannah.* Or ces neurotoxines CM et OH-26 ne sont pas décrites comme agissant sur les canaux ASIC ni présentant des effets analgésiques *in vivo* mais plutôt comme présentant des effets cytotoxiques. Il semble donc qu'en dessous de 55% d'identité de séquence avec ASICalgine-1 ou ASICalgine-2, les propriétés biologiques présentées par les peptides de l'invention soient perdues. En revanche, il est connu de l'art que des toxines isoformes et/ou orthologues présentent une activité conservée malgré quelques différences dans leur séquences protéique résultant en une identité de séquence de l'ordre de 70%. Il semble donc que des toxines isoformes et/ou orthologues présentant une identité de séquence d'au moins 56 à 70% avec ASICalgine-1 ou ASICalgine-2 conserveront les propriétés desdits peptides de l'invention.

### EXEMPLE 2 : EFFET ANALGESIQUE DES PEPTIDES ASICALGINE-1 (π-Dp1) ET ASICALGINE-2 (π-Dp2) IN VIVO APRES INJECTION INTRATHECALE

Le comportement douloureux des souris (C57B16J, mâles de 7 à 11 semaines) a été évalué après injection intrathécale (IT) des peptides entre les vertèbres L5 et L6, selon le protocole de Hylden et Wilcox [1980] [35]. Les effets d'une injection IT de 10µl de solution contenant 34 µM d'ASICalgine-1 (0,34 nmole) ou de 20 µM d'ASIcalgine-2 (0,2 nmole) ont été comparés avec les effets d'une injection IT de 10 µl de solution contenant 3,1 mM de morphine (31 nmole) ou de 10 µl de solution saline véhicule (145 mM NaCl, 5 mM KCI, 2 mM CaCl₂, 10 mM HEPES, pH 7,4, 0,05% albumine sérique bovine). Dans certaines séries d'expériences, la naxolone, un inhibiteur des récepteurs aux opioïdes (surtout de type µl), a été injectée par voie sous-cutanée à 2 mg/kg dans 50 µl de NaCl 0,9%, dix minutes avant l'injection IT du peptide ASICalgine.

### Toxicité

L'injection intrathécale des peptides ASICalgine-1 et ASICalgine-2 n'induit aucune modification du comportement de type troubles moteurs, troubles de l'équilibre, apathie, paralysie ou convulsions, ni aucun décès chez la souris.

### Douleur chimique aiguë et inflammatoire

L'injection sous-cutanée de formol (15 µl à 2% dans une solution de NaCl 0,9%) dans la voute plantaire de la patte arrière d'une souris induit une douleur présentant deux phases : une phase I de douleur chimique aiguë pendant 10 minutes et une phase Il de douleur inflammatoire de 15 à 45 minutes (Figure 2A).

La douleur est mesurée en observant le comportement douloureux spontané de l'animal, c'est-à-dire en comptant le temps pendant lequel la souris lève la patte injectée, la lèche, la mordille ou la secoue (comportement volontaire intégré).

L'injection IT d'ASICalgine-1 réduit significativement de 40% la douleur de la phase I et de 70% la douleur de la phase II. Cet effet analgésique est similaire à celui de la morphine (Figure 2B).

L'injection IT d'ASICalgine-2 produit un effet identique à ASICalgine-1.

Le prétraitement à la naxolone, un inhibiteur des récepteurs aux opiacés n'a aucun effet sur l'effet analgésique d'ASICalgine-1, que ce soit sur l'une ou l'autre des deux phases douloureuses I et II (Figure 2B).

Les peptides ASICalgine-1 et ASICalgine-2 sont les premiers peptides analgésiques extraits du venin de *Dendroaspis polylepis.*

### Douleur thermique aiguë

La douleur thermique a été étudiée chez la souris en mesurant la latence de retrait de la queue plongée dans de l'eau à 46°C (mouvement réflexe, l'animal est soumis à une contention), avec une durée limite de 30 s.

Le peptide ASICalgine-1 induit un effet analgésique aussi puissant que celui de la morphine (7 minutes après l'injection IT) mais qui est plus transitoire à la concentration utilisée (Figure 2C).

L'effet analgésique d'ASICalgine-1 n'est pas significativement inhibé par un prétraitement à la naxolone (Figure 2D).

### Hyperalgésie inflammatoire

L'injection sous-cutanée de carragénine 2% dans la voute plantaire de la patte arrière des souris, entraîne le développement d'une inflammation tissulaire et d'une hyperalgésie, mesurée par la latence de retrait de la patte arrière immergée dans de l'eau à 46°C (mouvement volontaire intégré, l'animal est soumis à une contention) avec une durée limite de 30 s, deux heures après l'injection de carragénine (Figure 2E, #).

L'injection IT d'ASICalgine-1 entraine une analgésie rapide, comparable à celle de la morphine et persistant toute la durée de l'expérience (67 minutes, Figure 2E).

L'effet analgésique d'ASICalgine-1 n'est que légèrement inhibé de 20% par un prétraitement à la naxolone (Figure 2F).

### Activité motrice

Les effets du peptide ASICalgine-1 sur l'activité motrice ont été évalués grâce au test du Rotarod accéléré. Les souris ont été placées sur un axe tournant à 4 rotations par minute (rpm) qui subit une accélération constante de 5 rpm par minute jusqu'à 40 rpm. La latence de la chute est mesurée, avec une limite maximale de 300 s.

L'injection IT du peptide ASICalgine-1 n'induit aucun effet significatif (test ANOVA) sur les performances des souris quant à l'activité motrice par rapport à une injection de solution véhicule.

### EXEMPLE 3 : EFFET ANALGESIQUE DU PEPTIDE ASICALGINE-1 (π-Dp1) IN VIVO APRES INJECTION INTRACEREBROVENTRICULAIRE

L'injection intracérébroventriculaire (ICV) du peptide ASICalgine-1 a été réalisée dans le troisième ventricule du cerveau de souris (C57B16J, mâles de 7 à 11 semaines) anesthésiées à l'isoflurane (1,5%) grâce aux coordonnées stéréotaxiques suivantes : -2,4 mm sous la surface corticale, -0,5 mm dans l'axe antéro-postérieur et +1,6 mm dans l'axe médio-latéral par rapport au bregma. Les effets d'une injection ICV de 5 µl de solution contenant 34 µM d'ASICalgine-1 (0,34 nmole) ont été comparés avec les effets de 5 µl de solution saline véhicule (145 mM NaCl, 5 mM KCI, 2 mM CaCl₂, 10 mM HEPES, pH 7,4, 0,05% sérum albumine bovine). La naxolone, un inhibiteur des récepteurs aux opioïdes (surtout µ), a été injectée par voie sous-cutanée à 2 mg/kg dans 50 µl de NaCl 0,9%, dix minutes avant l'injection ICV du peptide ASICalgine-1.

### Toxicité

L'injection ICV du peptide ASICalgine-1 n'induit aucune modification du comportement de type troubles moteurs, troubles de l'équilibre, apathie, paralysie ou convulsions, ni aucun décès chez les souris, même après 3 jours.

### Douleur thermique aigüe

La douleur thermique a été étudiée chez la souris en mesurant la latence de retrait de la queue plongée dans de l'eau à 46°C (mouvement réflexe, l'animal est soumis à une contention) avec une durée limite de 30 s.

L'injection ICV du peptide ASICalgine-1 induit un effet analgésique puissant et soutenu durant toute la durée de l'expérience (67 min, Figure 3), effet qui n'est pas modifié par un prétraitement à la naloxone. L'effet analgésique persiste plusieurs heures avant que la valeur de la latence de retrait de la queue ne retourne à sa valeur contrôle.

### EXEMPLE 4 : EFFET ANALGESIQUE DU PEPTIDE ASICALGINE-1 (π-Dp1) IN VIVO APRES INJECTION SOUS-CUTANEE

L'effet d'une injection sous-cutanée (SC) du peptide ASICalgine-1 a été testé sur l'hyperalgésie inflammatoire induite par la carragénine.

L'injection SC de carragénine 2% dans la voute plantaire de la patte arrière des souris (C57B16J, mâles de 7 à 11 semaines), entraîne le développement d'une inflammation tissulaire et d'une hyperalgésie, mesurée par la latence de retrait de la patte arrière immergée dans de l'eau à 46°C (mouvement volontaire intégré, l'animal est soumis à une contention) avec une durée limite de 30 s, deux heures après l'injection de carragénine.

Lorsque le peptide ASICalgine-1 (10 µl de solution contenant 34 µM d'ASICalgine-1 (0,34 nmole) dans du NaCl 0,9%, 0,05% albumine sérique bovine) est co-injecté avec la carragénine, aucune hyperalgésie inflammatoire thermique n'est constatée (Figure 4, □). Dans ces conditions, une seconde injection SC du peptide ASICalgine-1 deux heures après ne produit aucun effet significatif supplémentaire (Figure 4, □).

Si le peptide ASICalgine-1 n'a pas été co-injecté avec la carragénine, son injection SC lorsque l'inflammation est déjà installée, deux heures après l'injection de carragénine seule, réverse l'hyperalgésie inflammatoire et produit de plus une analgésie significative par rapport à la condition contrôle, avant l'injection de carragénine (Figure 4, ■ et #).

### EXEMPLE 5: ACTIVITE DES PEPTIDES ASICALGINE-1 (π-Dp1) ET ASICALGINE-2 (π-Dp2) SUR LES COURANTS ASIC

Les peptides ASICalgine-1 et ASICalgine-2 inhibent les courants générés par les canaux ASIC exprimés de façon hétérologue dans la lignée cellulaire COS et enregistrés par la technique du patch-clamp en configuration « cellule entière » (mode « potentiel-imposé »).

Les peptides sont appliqués à pH 7,4, 30 secondes avant le saut de pH extracellulaire de 7,4 à 6 ou 5,5.

Les peptides ASICalgine-1 et ASICalgine-2 ont le même effet, et inhibent le courant généré par les canaux homomériques ASIC1a de rat avec une concentration produisant 50% d'inhibition (CI₅₀) de 49 nM (Figure 5A), et le courant généré par les canaux homomériques ASIC1a humain avec une CI₅₀ de 127nM (Figure 5B). Le courant généré par les canaux homomériques ASIC1b de rat est inhibé avec une CI₅₀ de 650 nM (Figure 5C), les canaux hétéromériques ASIC1 a+ASIC2a de rat et humain avec une CI₅₀ de 308 nM (Figure 5E), et les canaux hétéromériques ASIC1 a+ASIC1 b de rat avec une CI₅₀ de 223 nM (Figure 5D) (le canal ASIC1b n'a pas été décrit chez l'homme).

Les peptides ASICalgine-1 et ASICalgine-2 n'inhibent pas les canaux homomériques ASIC2a et ASIC3 de rat et humain, ni les canaux hétéromériques ASIC1 a+ASIC3 et ASIC1b+ASIC3 de rat.

Les effets des peptides ASICalgine-1 et ASICalgine-2 sont réversibles à l'arrêt de l'application des peptides (Figure 5).

Les peptides ASICalgine-1 et ASICalgine-2 sont les seuls peptides décrits à ce jour capables d'inhiber les canaux homomériques ASIC1b et les canaux hétéromériques ASIC1 a+ASIC1 b et ASIC1 a+ASIC2a.

Par contre, le peptide ASICalgine-1 (2 µM) n'inhibe pas le courant produit par les canaux TRPV1 de rat activés par la capsaïcine (1 µM), canaux également impliqués dans la transduction de la douleur par les terminaisons nerveuses sensorielles.

Sans se trouver limité à cette explication, les effets analgésiques des peptides ASICalgine-1 et ASICalgine-2 apparaissent comme mettant en jeu l'inhibition des canaux ASIC1a homomériques et ASIC1a+ASIC2a hétéromériques dans les neurones du système nerveux central (injections IT et ICV), et l'inhibition des canaux ASIC1a et ASIC1b homomériques et ASIC1a+ASIC1b hétéromériques dans les neurones sensoriels (injection sous-cutanée, injection IT sous réserve que ASIC1a et ASIC1b homomériques et ASIC1a+ASIC1b hétéromériques soient présents dans les terminaisons centrales des neurones sensoriels au niveau de la corne dorsale de la moelle épinière).

### EXEMPLE 6: ACTIVITE DES PEPTIDES ASICALGINE-1 (π-Dp1) ET ASICALGINE-2 (π-Dp2) SUR LES COURANTS ASIC NATIFS DES NEURONES CENTRAUX ET DES NEURONES SENSORIELS

Les peptides ASICalgine-1 et ASICalgine-2 inhibent de façon réversible les courants ASIC enregistrés par la technique du patch-clamp en configuration « cellule entière » (mode « potentiel-imposé ») dans des neurones en culture primaire isolés à partir de ganglions rachidiens de rat adulte (Figure 6A), de moelle épinière dorsale d'embryons de souris (Figure 6B), et d'hippocampe de nouveaux-nés de souris (2 jours) (Figure 6C).

L'effet inhibiteur partiel des peptides ASICalgine-1 et ASICalgine-2 sur le courant ASIC total des neurones sensoriels s'explique par la part importante de courant de type ASIC3 (non inhibé par les peptides ASICalgine-1 et ASICalgine-2) dans les neurones sensoriels, alors que les courants ASIC centraux (hippocampe et moelle) sont composés d'un mélange de courants homomérique ASIC1a et hétéromérique ASIC1a+ASIC2a (tous deux inhibés par les peptides ASICalgine-1 et ASICalgine-2).

### EXEMPLE 7: ACTIVITE DES PEPTIDES ASICALGINE-1 (π-Dp1) ET ASICALGINE-2 (π-Dp2) SUR LES NEURONES

Les peptides ASICalgine-1 et ASICalgine-2 appliqués sur les neurones spinaux en culture, n'induisent aucune modification du niveau de courant basal, ni de variations du potentiel de repos (Figure 7A) enregistrés par la technique du patch-clamp en configuration « cellule entière ».

L'application des peptides ASICalgine-1 et ASICalgine-2 ne modifie pas l'activité électrique synaptique spontanée des neurones spinaux en culture, ni la forme ou le seuil de potentiels d'action évoqués par une stimulation électrique (mode « courant-imposé » du patch-clamp) (Figure 7B).

Les résultats montrent que les peptides ASICalgine-1 et ASICalgine-2 n'ont pas d'effet sur les propriétés d'excitabilité intrinsèques des neurones, sur la fonction synaptique ou sur les courants dépendants du potentiel impliqués dans la genèse (canaux Na⁺) ou la repolarisation (canaux K⁺) des potentiels d'action.

En revanche, l'inhibition spécifique des canaux ASIC par les peptides ASICalgine-1 et ASICalgine-2 réduit l'excitabilité des neurones en réponse à un saut de pH acide (de 7,4 à 6,0), la dépolarisation produite par le courant ASIC n'étant alors pas suffisante pour déclencher de potentiel d'action (Figure 7A).

### Liste de références

1. Bertin et Vergne-Salle, « Traitements médicamenteux de l'inflammation et des douleurs inflammatoires » Institut UPSA de la douleur-A Editorial paris, p.113-132, 2007
2. Gutstein et Akil, "Opioid analgesics", in "The Pharmacological Basis of Therapeutics" Brunton et al., Eds. Mc Graw-Hill, 2006.
3. Bannister & Dickenson, Curr. Opin. Support Palliat. Care, 4 : 1-5, 2010
4. Yennurajalingam et al., Support Cancer Ther., 1: 97-110, 2004
5. Mizoguchi et al., Int. Rev. Neurobiol., 85: 249-260, 2009
6. Waldmann et Lazdunski, Curr. Opin. Neurobiol., 3 : 418-424, 1998
7. Wemmie et al., Trends Neurosci., 29 : 578-586, 2006
8. Lingueglia et al., J. Biol. Chem., 282 : 17325-17329, 2007
9. Jasti et al., Nature, 449 : 316-323, 2007
10. Lingueglia et al., J. Biol. Chem., 272 : 29778-29783, 1997
11. Benson et al., Proc. Natl. Acad. Sci. U.S.A., 95 : 10240-10245, 2002
12. Hesselager et al., J. Biol. Chem., 279 : 11006-11015, 2004
13. Waldmann et al., Nature, 386 : 173-177, 1997a
14. Noel et al., « Current perspectives on acid-sensing ion channels : new advances and therapeuthic implications » Expert Rev. Clin. Pharmacol. « Clinical Pharmacology of Ion Channels », 3 : 331-346, 2010
15. Waldmann et al., J. Biol. Chem., 272 : 20975-20978, 1997b
16. Bassler et al., J. Biol. Chem., 276 : 33782-33787, 2001
17.Chen et al., Proc. Natl. Adad. Sci. U.S.A., 95 : 10240-10245, 1998
18.Reeh et Steen, Prog. Brain Res., 113 : 143-151, 19961996
19.Steen et al., Neurosci. Lett., 199 : 29-32, 1995a
20. Issberner et al., Neurosci. Lett., 208 : 191-194, 1996
21. Ugawa et al., J. Clin. Invest., 10 : 1185-1191, 2002
22.Jones et al., J. Neurosci., 24 : 10974-10979, 2004
23. Voilley et al., J. Neurosci., 21 : 8026-8033, 2001
24.Dubé et al., Pain, 117 : 88-96, 2005
25. Escoubas et al., J. Biol. Chem., 275 : 25116-25121, 2000
26. Diochot et al., EMBO J., 23 : 1516-1525, 2004
27. Deval et al., EMBO J., 27: 3047-3055, 2008
28. Mazzuca et al., Nature Neuroscience, 10 : 943-945, 2007
29.Abott et al., Pharmacol. Biochem. Behav., 17(6) : 1213-1219, 1982
30.Cridland et Henry, Brain Res., 584(1-2) : 163-168, 1992
31. Woolfe et Macdonald, J. Pharmacol. Exp. Ther., 80 : 300-307, 1944
32.Ankier, Eur. J. Pharmacol., 27(1) : 1-4, 1974
33. Kim et al., Pain, 55 : 85-92, 1993
34. Brevet US 5,877,026
35. Hylden et Wilcox, Eur. J. Pharmacol., 67 : 313-316, 1980
36. Deval et al., J. Neurosci., 31(16) : 6059-6066, 2011

## Revendications

1. Peptide comprenant la séquence en acides aminés où X⁴ représente n'importe quel acide aminé ; ou une séquence présentant une identité d'au moins 56% avec la séquence SEQ ID NO: 1 et conservant une activité analgésique dudit peptide comprenant la séquence SEQ ID NO : 1.

2. Peptide selon la revendication 1, où ledit peptide est un bloqueur d'au moins un canal ASIC.

3. Peptide selon la revendication 2, où ledit peptide est un bloqueur d'au moins un canal ASIC contenant au moins une sous-unité choisie dans le groupe constitué des sous-unités ASIC1a et ASIC1b.

4. Peptide selon la revendication 3, où ledit peptide est un bloqueur du canal homomérique ASIC1a, homomérique ASIC1b, hétéromérique ASIC1a+ASIC1b et/ou hétéromérique ASIC1a+ASIC2a.

5. Peptide selon l'une quelconque des revendications 1 à 4, où X⁴ représente Y ou F dans la séquence SEQ ID NO : 1.

6. Polynucléotide comprenant une séquence en nucléotides codant pour un peptide tel que défini dans l'une quelconque des revendications 1 à 5.

7. Polynucléotide selon la revendication 6, comprenant la séquence en nucléotides : ou où ⁷³txx⁷⁵ et ¹⁰txx¹² représente tac, tat, ttt, ou ttc.

8. Polynucléotide selon la revendication 6, où ledit polynucléotide s'hybride en conditions stringentes avec la séquence en nucléotides SEQ ID NO : 4 ou SEQ ID NO : 22, ou une séquence complémentaire de celle-ci.

9. Polynucléotide selon la revendication 6 ou 8, où ledit polynucléotide comprend une séquence naturelle ou synthétique présentant une identité d'au moins 76% avec la séquence SEQ ID NO: 4 ou la séquence SEQ ID NO: 22.

10. Vecteur comprenant un polynucléotide tel que défini dans l'une quelconque des revendications 6 à 9.

11. Vecteur selon la revendication 10, où ledit vecteur est un vecteur d'expression.

12. Cellule hôte comprenant un vecteur tel que défini dans l'une quelconque des revendications 10 ou 11.

13. Composition pharmaceutique comprenant un ou plusieurs peptide tel que défini dans l'une quelconque des revendications 1 à 5, polynucléotide tel que défini dans l'une quelconque des revendications 6 à 9, vecteur tel que défini dans l'une quelconque des revendications 10 ou 11, ou cellule hôte telle que définie dans la revendication 12.

14. Substance choisie parmi :
- un peptide tel que défini dans l'une quelconque des revendications 1 à 5;
- un polynucléotide tel que défini dans l'une quelconque des revendications 6 à 9 ;
- un vecteur tel que défini dans l'une quelconque des revendications 10 ou 11 ; ou
- une cellule hôte telle que définie dans la revendication 12 ;
- une composition pharmaceutique selon la revendication 13 ;
pour son application comme médicament.

15. Substance selon la revendication 14, où ledit médicament est un analgésique.

16. Substance selon la revendication 15, où l'analgésique est destiné à la prévention ou au traitement d'une douleur ou d'une pathologie impliquant l'activation des canaux ASIC.

17. Substance selon la revendication 16, où la douleur et la pathologie impliquant l'activation des canaux ASIC est choisie dans le groupe comprenant les douleurs inflammatoires, neuropathiques, cancéreuses, post-opératoires, musculo-squelettiques et viscérales, les inflammations, les cancers, les fibromyalgies, et le syndrome du colon irritable.

18. Substance selon la revendication 14, où ledit médicament est destiné à la prévention ou au traitement d'une maladie neurologique centrale choisie dans le groupe comprenant la dépression, l'anxiété, les attaques cérébrales, l'épilepsie, les inflammations centrales et les maladies neurodégénératives.

19. Substance selon l'une quelconque des revendications 14 à 18, où ledit médicament est administré par voie centrale, sous-cutanée, trans-cutanée, systémique, orale, ou respiratoire.

20. Substance choisie parmi :
- un peptide tel que défini dans l'une quelconque des revendications 1 à 5;
- un polynucléotide tel que défini dans l'une quelconque des revendications 6 à 9 ;
- un vecteur tel que défini dans l'une quelconque des revendications 10 ou 11 ; ou
- une cellule hôte telle que définie dans la revendication 12 ;
- une composition pharmaceutique selon la revendication 13 ;
pour son application comme outil diagnostic.

21. Procédé d'identification d'un composé mimant l'activité analgésique d'un peptide tel que défini dans l'une quelconque des revendications 1 à 5, comprenant les étapes suivantes :
a. déterminer l'activité analgésique d'un peptide tel que défini dans l'une quelconque des revendications 1 à 5 ;
b. déterminer l'activité analgésique d'un composé candidat ;
c. comparer les activités analgésiques obtenues aux étapes a) et b) ;
d. sélectionner le composé candidat présentant une activité analgésique équivalente ou supérieure à celle dudit peptide.

22. Procédé d'identification d'un composé mimant l'activité analgésique d'un peptide tel que défini dans l'une quelconque des revendications 1 à 5, comprenant les étapes suivantes :
a) mettre en contact un peptide tel que défini dans l'une quelconque des revendications 1 à 5 avec un échantillon, et mesurer la liaison dudit peptide avec ledit échantillon ;
b) ajouter un composé candidat, et évaluer l'effet dudit composé sur la liaison dudit peptide avec ledit échantillon ;
c) sélectionner le composé candidat capable de moduler la liaison dudit peptide avec ledit échantillon.

## Patentansprüche

1. Peptid, das die Aminosäuresequenz wobei X⁴ eine beliebige Aminosäure; oder eine Sequenz darstellt, die eine Identität von mindestens 56% mit der Sequenz SEQ ID NO : 1 aufweist und die die analgetische Aktivität des die Sequenz SEQ ID NO : 1 umfassenden Peptids beibehält.

2. Peptid nach Anspruch 1, wobei das Peptid mindestens einen ASIC-Kanals blockiert.

3. Peptid nach Anspruch 2, wobei das Peptid mindestens einen ASIC-Kanals blockiert, der aus mindestens einer Untereinheit besteht, die aus der Gruppe bestehend aus den Untereinheiten ASIC1a und ASIC1b ausgewählt ist.

4. Peptid nach Anspruch 3, wobei das Peptid den homomerischen Kanal ASIC1a, den homomerischen Kanal ASIC1b, den heteromerischen Kanal ASIC1a+ASIC1b und/oder den heteromerischen Kanal ASIC1a+ASIC2a blockiert.

5. Peptid nach einem beliebigen der Ansprüche 1 bis 4, wobei X⁴ Y oder F in der Sequenz SEQ ID NO : 1 darstellt.

6. Polynucleotid eine Nucleotidsequenz umfassend, die für ein wie in einem beliebigen der Ansprüche 1 bis 5 definiertes Peptid kodiert.

7. Polynucleotid nach Anspruch 6, das folgende Nucleotidsequenz umfasst: oder wo⁷³txx⁷⁵ und ¹⁰txx¹² für tac, tat, ttt, oder ttc stehen.

8. Polynucleotid nach Anspruch 6, wobei das Polynucleotid sich unter stringenten Bedingungen mit der Nucleotidsequenz SEQ ID NO : 4 oder SEQ ID NO : 22 oder einer komplementären Sequenz von diesen hybridiert.

9. Polynucleotid nach Anspruch 6 oder 8, wobei das Polynucleotid eine natürliche oder synthetische Sequenz umfasst, die eine Identität von mindestens 76% mit der Sequenz SEQ ID NO: 4 oder der Sequenz SEQ ID NO: 22 aufweist.

10. Vektor, der ein Polynucleotid wie in einem beliebigen der Ansprüche 1 bis 5 definiert enthält.

11. Vektor nach Anspruch 10, wobei der Vektor ein Expressionsvektor ist.

12. Wirtszelle, die einen Vektor wie in einem der Ansprüche 10 oder 11 definiert umfasst.

13. Pharmazeutische Zusammensetzung umfassend ein oder mehrere Peptide wie in einem beliebigen der Ansprüche 1 bis 5 definiert, Polynucleotid wie in einem beliebigen der Ansprüche 6 bis 9 definiert, Vektor, wie einem beliebigen der Ansprüche 10 oder 11 definiert oder Wirtszelle wie in Anspruch 12 definiert.

14. Substanz ausgewählt unter:
- einem Peptid wie in einem beliebigen der Ansprüche 1 bis 5 definiert;
- einem Polynucleotid wie in einem beliebigen der Ansprüche 6 bis 9 definiert;
- einem Vektor wie in einem beliebigen der Ansprüche 10 oder 11 definiert; oder
- einer Wirtszelle wie in Anspruch 12 definiert;
- einer pharmazeutische Zusammensetzung nach Anspruch 13 ;
für ihre Verwendung als Medikament.

15. Substanz nach Anspruch 14, wobei das Medikament ein Analgetikum ist.

16. Substanz nach Anspruch 15, wobei das Analgetikm zur Vorbeugung oder Behandlung eines Schmerzes oder einer Pathologie ist, die eine Aktivierung der ASIC-Kanäle beinhaltet.

17. Substanz nach Anspruch 16, wobei der Schmerz oder die Pathologie mit einer Aktivierung der ASIC-Kanäle aus der Gruppe ausgewählt ist, die entzündliche, neuropathische Schmerzen, Krebsschmerzen, postoperative Schmerzen, Skelett-und Muskelschmerzen, viszerale Schmerzen, Entzündungen, Krebs, Fibromyalgien und das Reizdarmsyndrom umfasst.

18. Substanz nach Anspruch 14, wobei das Medikament zur Vorbeugung oder Behandlung einer zentralen neurologischen Krankheit bestimmt ist, die aus der Gruppe ausgewählt ist, die Depression, Angstzustände, Hirnschläge, Epilepsie, zentrale Entzündungen und neurodegenerative Krankheiten umfasst.

19. Substanz nach einem beliebigen der Ansprüche 14 bis 18, wobei das Medikament zentral, subkutan, transkutan, systemisch, oral oder respiratorisch verabreicht wird.

20. Substanz ausgewählt unter:
- einem Peptid wie in einem beliebigen der Ansprüche 1 bis 5 definiert;
- einem Polynucleotid wie in einem beliebigen der Ansprüche 6 bis 9 definiert;
- einem Vektor wie in einem der beliebigen Ansprüche 10 oder 11 definiert; oder
- einer Wirtszelle wie in Anspruch 12 definiert;
- einer pharmazeutische Zusammensetzung nach Anspruch 13 ;
für ihre Anwendung als diagnostisches Werkzeug.

21. Verfahren zur Identifizierung einer Verbindung, die die analgetische Aktivität eines Peptids wie in einem beliebigen der Ansprüche 1 bis 5 definiert nachahmt, welches folgende Schritte umfasst:
a. Bestimmung der analgetischen Wirkung eines Peptids wie in einem beliebigen der Ansprüche 1 bis 5 definiert;
b. Bestimmung der analgetischen Wirkung einer Kandidatenverbindung ;
c. Vergleich der in den Schritten a) und b) erhaltenen analgetischen Wirkung;
d. Auswahl der Kandidatenverbindung, die eine gleichwertige oder grössere analgetische Aktivität als das Peptid aufweist.

22. Verfahren zur Identifizierung einer Verbindung, die die analgetische Wirkung eines Peptids wie einem beliebigen der Ansprüche 1 bis 5 definiert nachahmt, welches folgende Schritte umfasst:
a) in Kontakt bringen eines Peptids wie in einem beliebigen der Ansprüche 1 bis 5 definiert mit einer Probe und Messen der Bindung zwischen dem Peptid und der Probe;
b) Hinzufügen der Kandidatenverbindung und Bestimmen der Wirkung der Verbindung auf die Bindung zwischen dem Peptid und der Probe ;
c) Auswahl der Kandidatenverbindung, welche die Bindung zwischen dem Peptid und der Probe modulieren kann.

## Claims

1. Peptide comprising the amino acid sequence wherein X⁴ represents any amino acid; or a sequence exhibiting an identity of at least 56% with the sequence SEQ ID No: 1 and retaining the analgesic activity of said peptide comprising the sequence SEQ ID No: 1.

2. Peptide as claimed in claim 1, wherein said peptide is a blocker of at least an ASIC channel.

3. Peptide as claimed in claim 2, wherein said peptide is a blocker of at least an ASIC channel containing at least a subunit chosen in the group consisting of ASIC1a and ASIC1b subunits.

4. Peptide as claimed in claim 3, wherein said peptide is a blocker of the homomeric ASIC1a channel, homomeric ASIC1b channel, heteromeric ASIC1a+ASIC1b channel and/or heteromeric ASIC1a+ASIC2a channel.

5. Peptide as claimed in any one of claims 1 to 4, wherein X⁴ represents Y or F in the sequence SEQ ID No: 1.

6. Polynucleotide comprising a nucleotide sequence encoding a peptide as defined in any one of claims 1 to 5.

7. Polynucleotide as claimed in claim 6, comprising the nucleotide sequence: or wherein ⁷³txx⁷⁵ and ¹⁰txx¹² represent tac, tat, ttt or ttc.

8. Polynucleotide as claimed in claim 6, wherein said polynucleotide hybridizes under stringent conditions with the nucleotide sequence SEQ ID No: 4 or SEQ ID No: 22, or a sequence complementary thereto.

9. Polynucleotide as claimed in claim 6 or 8, wherein said polynucleotide comprises a natural or synthetic sequence exhibiting an identity of at least 76% with the sequence SEQ ID No: 4 or the sequence SEQ ID No: 22.

10. Vector comprising a polynucleotide as defined in any one of claims 6 to 9.

11. Vector as claimed in claim 10, wherein said vector is an expression vector.

12. Host cell comprising a vector as defined in either one of claims 10 or 11.

13. Pharmaceutical composition comprising one or more peptides as defined in any one of claims 1 to 5, polynucleotides as defined in any one of claims 6 to 9, vectors as defined in either one of claims 10 or 11, or host cells as defined in claim 12.

14. Substance chosen from:
- a peptide as defined in any one of claims 1 to 5;
- a polynucleotide as defined in any one of claims 6 to 9;
- a vector as defined in either one of claims 10 or 11; or
- a host cell as defined in claim 12;
- a pharmaceutical composition as claimed in claim 13;
for use as a medicament.

15. Substance as claimed in claim 14, wherein said medicament is an analgesic.

16. Substance as claimed in claim 15, wherein the analgesic is intended for the prevention or treatment of pain or of a pathological condition involving the activation of ASIC channels.

17. Substance as claimed in claim 16, wherein the pain and the pathological condition involving the activation of ASIC channels are chosen from the group comprising inflammatory, neuropathic, cancer-related, postoperative, musculoskeletal and visceral pain, inflammations, cancers, fibromyalgia and irritable bowel syndrome.

18. Substance as claimed in claim 16, wherein said medicament is intended for the prevention or treatment of a central neurological disease chosen from the group comprising depression, anxiety, strokes, epilepsy, central inflammations and neurodegenerative diseases.

19. Substance as claimed in any one of claims 14 to 18, wherein said medicament is administered centrally, subcutaneously, transcutaneously, systemically, orally or via the respiratory route.

20. Substance chosen from:
- a peptide as defined in any one of claims 1 to 5;
- a polynucleotide as defined in any one of claims 6 to 9;
- a vector as defined in either one of claims 10 and 11; or
- a host cell as defined in claim 12;
- a pharmaceutical composition as claimed in claim 14;
for use as a diagnostic tool.

21. Method for identifying a compound which mimics the analgesic activity of a peptide as defined in any one of claims 1 to 5, comprising the following steps:
a. determining the analgesic activity of a peptide as defined in any one of claims 1 to 5;
b. determining the analgesic activity of a candidate compound;
c. comparing the analgesic activities obtained in steps a. and b.;
d. selecting the candidate compound which has an analgesic activity equivalent to or greater than that of said peptide.

22. Method for identifying a compound which mimics the analgesic activity of a peptide as defined in any one of claims 1 to 5, comprising the following steps:
a) bringing a peptide as defined in any one of claims 1 to 5 into contact with a sample, and measuring the binding of said peptide with said sample;
b) adding a candidate compound, and evaluating the effect of said compound on the binding of said peptide with said sample;
c) selecting the candidate compound capable of modulating the binding of said peptide with said sample.


### SEQUENCE LISTING

<110> Centre National de la Recherche Scientifique (CNRS) LINGUEGLIA, Eric DIOCHOT, Sylvie BARON-FORSTER, Anne SALINAS, Miguel LAZDUNSKI, Michel
<120> NOUVEAUX PEPTIDES AVEC DES EFFETS ANALGESIQUES ET INHIBANT LES CANAUX ASIC
<130> BIP206637PC00
<150> FR 1003136
   <151> 2010-07-26
<160> 22
<170> PatentIn version 3.5
<210> 1
   <211> 57
   <212> PRT
   <213> Dendroaspis polylepis
<220>
   <221> misc_feature
   <222> (4)..(4)
   <223> Xaa can be any naturally occurring amino acid
<400> 1
   Leu Lys Cys Xaa Gln His Gly Lys Val Val Thr Cys His Arg Asp Met
   1               5                   10                  15
   Lys Phe Cys Tyr His Asn Thr Gly Met Pro Phe Arg Asn Leu Lys Leu
               20                  25                  30
   Ile Leu Gln Gly Cys Ser Ser Ser Cys Ser Glu Thr Glu Asn Asn Lys
           35                  40                  45
   Cys Cys Ser Thr Asp Arg Cys Asn Lys
       50                  55
<210> 2
   <211> 57
   <212> PRT
   <213> Dendroaspis polylepis
<400> 2
   Leu Lys Cys Tyr Gln His Gly Lys Val Val Thr Cys His Arg Asp Met
   1               5                   10                  15
   Lys Phe Cys Tyr His Asn Thr Gly Met Pro Phe Arg Asn Leu Lys Leu
               20                  25                  30
   Ile Leu Gln Gly Cys Ser Ser Ser Cys Ser Glu Thr Glu Asn Asn Lys
           35                  40                  45
   Cys Cys Ser Thr Asp Arg Cys Asn Lys
       50                  55
<210> 3
   <211> 57
   <212> PRT
   <213> Dendroaspis polylepis
<400> 3
   Leu Lys Cys Phe Gln His Gly Lys Val Val Thr Cys His Arg Asp Met
   1               5                   10                  15
   Lys Phe Cys Tyr His Asn Thr Gly Met Pro Phe Arg Asn Leu Lys Leu
               20                  25                  30
   Ile Leu Gln Gly Cys Ser Ser Ser Cys Ser Glu Thr Glu Asn Asn Lys
           35                  40                  45
   Cys Cys Ser Thr Asp Arg Cys Asn Lys
       50                  55
<210> 4
   <211> 237
   <212> DNA
   <213> Dendroaspis polylepis
<220>
   <221> misc_feature
   <222> (74)..(74)
   <223> n correspond à a ou t
<220>
   <221> misc_feature
   <222> (75)..(75)
   <223> n correspond à c ou t
<400> 4
   atgaaaactc tgctgctgac cttgctggtg gtgacaatcg tgtgcctaga cttaggatac     60
   tccctgaaat gttnncaaca tggtaaagtt gtgacttgtc atcgagatat gaagttttgc    120
   tatcataaca ctggcatgcc ttttcgaaat ctcaagctca tcctacaggg atgttcttct    180
   tcgtgcagtg aaacagaaaa caataagtgt tgctcaacag acagatgcaa caaatag       237
<210> 5
   <211> 446
   <212> DNA
   <213> Dendroaspis polylepis
<220>
   <221> CDS
   <222> (1)..(237)
<220>
   <221> sig_peptide
   <222> (1)..(63)
<220>
   <221> polyA_site
   <222> (446)..(446)
<400> 5
   atg aaa act ctg ctg ctg acc ttg ctg gtg gtg aca atc gtg tgc cta        48
   Met Lys Thr Leu Leu Leu Thr Leu Leu Val Val Thr Ile Val Cys Leu
   1               5                   10                  15
   gac tta gga tac tcc ctg aaa tgt tac caa cat ggt aaa gtt gtg act        96
   Asp Leu Gly Tyr Ser Leu Lys Cys Tyr Gln His Gly Lys Val Val Thr
               20                  25                  30
   tgt cat cga gat atg aag ttt tgc tat cat aac act ggc atg cct ttt        144
   Cys His Arg Asp Met Lys Phe Cys Tyr His Asn Thr Gly Met Pro Phe
           35                  40                  45
   cga aat ctc aag ctc atc cta cag gga tgt tct tct tcg tgc agt gaa        192
   Arg Asn Leu Lys Leu Ile Leu Gln Gly Cys Ser Ser Ser Cys Ser Glu
       50                  55                  60
   aca gaa aac aat aag tgt tgc tca aca gac aga tgc aac aaa tag            237
   Thr Glu Asn Asn Lys Cys Cys Ser Thr Asp Arg Cys Asn Lys
   65                  70                  75
   ctctacgagt ggctaaattc attgagtttt gctctcatcc attgtggacc atccttgaaa      297
   atttatgctt gtggccttta ccaccagatg gtccatcatc cccctctccc ctgctttctt      357
   tgatacctca tcatctttcc cttttctctt gttctgtaat ttccttctgc tagttctgta      417
   gtttgagaat caaataaaac tcagcattc                                        446
<210> 6
   <211> 78
   <212> PRT
   <213> Dendroaspis polylepis
<400> 6
   Met Lys Thr Leu Leu Leu Thr Leu Leu Val Val Thr Ile Val Cys Leu
   1               5                   10                  15
   Asp Leu Gly Tyr Ser Leu Lys Cys Tyr Gln His Gly Lys Val Val Thr
               20                  25                  30
   Cys His Arg Asp Met Lys Phe Cys Tyr His Asn Thr Gly Met Pro Phe
           35                  40                  45
   Arg Asn Leu Lys Leu Ile Leu Gln Gly Cys Ser Ser Ser Cys Ser Glu
       50                  55                  60
   Thr Glu Asn Asn Lys Cys Cys Ser Thr Asp Arg Cys Asn Lys
   65                  70                  75
<210> 7
   <211> 20
   <212> DNA
   <213> Artificial Sequence
<220>
   <223> Oigonucléotide
<220>
   <221> misc_feature
   <222> (3)..(3)
   <223> n correspond à la base inosine capable de s'hybrider avec a, t, g ou c
<220>
   <221> misc_feature
   <222> (6)..(6)
   <223> y peut être t ou c
<220>
   <221> misc_feature
   <222> (9)..(9)
   <223> r peut être g ou a
<220>
   <221> misc_feature
   <222> (12)..(12)
   <223> y peut être t ou c
<220>
   <221> misc_feature
   <222> (15)..(15)
   <223> n correspond à la base inosine capable de s'hybrider avec a, t, g ou c
<220>
   <221> misc_feature
   <222> (18)..(18)
   <223> r peut être g ou a
<400> 7
   tgnttycarc ayggnaargt 20
<210> 8
   <211> 21
   <212> DNA
   <213> Artificial Sequence
<220>
   <223> Oligonucléotide
<220>
   <221> misc_feature
   <222> (1)..(1)
   <223> y peut être t ou c
<220>
   <221> misc_feature
   <222> (4)..(4)
   <223> n correspond à la base inosine capable de s'hybrider avec a, t, g ou c
<220>
   <221> misc_feature
   <222> (6)..(7)
   <223> r peut être g ou a
<220>
   <221> misc_feature
   <222> (10)..(10)
   <223> n correspond à la base inosine capable de s'hybrider avec a, t, g ou c
<220>
   <221> misc_feature
   <222> (13)..(13)
   <223> r peut être g ou a
<220>
   <221> misc_feature
   <222> (16)..(16)
   <223> n correspond à la base inosine capable de s'hybrider avec a, t, g ou c
<400> 8
   yttnarrttn cgraanggca t 21
<210> 9
   <211> 30
   <212> DNA
   <213> Artificial Sequence
<220>
   <223> Oligonucléotide
<400> 9
   acacgaattc gctatcataa cactggcatg 30
<210> 10
   <211> 40
   <212> DNA
   <213> Artificial Sequence
<220>
   <223> Oligonucléotide
<400> 10
   acacgaattc tttttttttt tttttttttt tttttttttt 40
<210> 11
   <211> 31
   <212> DNA
   <213> Artificial Sequence
<220>
   <223> Oligonucléotide
<400> 11
   acacgaattc tccagagaag atcgcaagat g 31
<210> 12
   <211> 31
   <212> DNA
   <213> Artificial Sequence
<220>
   <223> Oligonucléotide
<400> 12
   acacgaattc atttagccac tcgtagagct a 31
<210> 13
   <211> 6
   <212> PRT
   <213> Dendroaspis polylepis
<400> 13
   Cys Phe Gln His Gly Lys
   1               5
<210> 14
   <211> 6
   <212> PRT
   <213> Dendroaspis polylepis
<400> 14
   Cys Tyr Gln His Gly Lys
   1               5
<210> 15
   <211> 12
   <212> PRT
   <213> Dendroaspis polylepis
<400> 15
   Cys Phe Gln His Gly Lys Val Val Thr Cys His Arg
   1               5                   10
<210> 16
   <211> 12
   <212> PRT
   <213> Dendroaspis polylepis
<400> 16
   Cys Tyr Gln His Gly Lys Val Val Thr Cys His Arg
   1               5                   10
<210> 17
   <211> 6
   <212> PRT
   <213> Dendroaspis polylepis
<400> 17
   Val Val Thr Cys His Arg
   1 5
<210> 18
   <211> 14
   <212> PRT
   <213> Dendroaspis polylepis
<400> 18
   Asp Met Lys Phe Cys Tyr His Asn Thr Gly Met Pro Phe Arg
   1 5 10
<210> 19
   <211> 17
   <212> PRT
   <213> Dendroaspis polylepis
<400> 19
   Leu Ile Leu Gln Gly Cys Ser Ser Ser Cys Ser Glu Thr Glu Asn Asn
   1 5 10 15
   Lys
<210> 20
   <211> 6
   <212> PRT
   <213> Dendroaspis polylepis
<400> 20
   Cys Cys Ser Thr Asp Arg
   1 5
<210> 21
   <211> 23
   <212> PRT
   <213> Dendroaspis polylepis
<400> 21
   Leu Ile Leu Gln Gly Cys Ser Ser Ser Cys Ser Glu Thr Glu Asn Asn
   1 5 10 15
   Lys Cys Cys Ser Thr Asp Arg
   20
<210> 22
   <211> 174
   <212> DNA
   <213> Dendroaspis polylepis
<220>
   <221> misc_feature
   <222> (11)..(11)
   <223> n correspond à a ou t
<220>
   <221> misc_feature
   <222> (12)..(12)
   <223> n correspond à c ou t
<400> 22
   ctgaaatgtt nncaacatgg taaagttgtg acttgtcatc gagatatgaa gttttgctat        60
   cataacactg gcatgccttt tcgaaatctc aagctcatcc tacagggatg ttcttcttcg       120
   tgcagtgaaa cagaaaacaa taagtgttgc tcaacagaca gatgcaacaa atag             174